(19) Europäisches Patentamt

European Patent Office

Office européen des brevets

(11) **EP 3 021 805 B1**

(12) **EUROPEAN PATENT SPECIFICATION**

(45) Date of publication and mention
of the grant of the patent:
**20.04.2022 Bulletin 2022/16**

(21) Application number: **13742190.5**

(22) Date of filing: **19.07.2013**

(51) International Patent Classification (IPC):
***A61F 13/00*** *(2006.01)*        ***A61F 13/08*** *(2006.01)*
***D04B 21/18*** *(2006.01)*

(52) Cooperative Patent Classification (CPC):
**A61F 13/00038; A61F 13/08; D04B 21/18;**
A61F 2013/00131; D10B 2509/028

(86) International application number:
**PCT/EP2013/065281**

(87) International publication number:
**WO 2015/007335 (22.01.2015 Gazette 2015/03)**

(54) **ELASTIC BANDAGE**

ELASTISCHE BANDAGE

BANDE ÉLASTIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**25.05.2016 Bulletin 2016/21**

(73) Proprietor: **Presscise AB
524 30 Herrljunga (SE)**

(72) Inventors:
• **LUNDH, Torbjörn
S-427 35 Billdal (SE)**
• **MATTSSON, Erney
S-426 76 Västra Frölunda (SE)**
• **VASILIS, Jonathan
S-411 29 Göteborg (SE)**

(74) Representative: **Lind Edlund Kenamets
Intellectual Property AB
Östra Hamngatan 17
411 10 Göteborg (SE)**

(56) References cited:
**EP-A1- 1 785 267        WO-A2-2010/049620
GB-A- 2 104 558        US-A- 6 050 967
US-A1- 2009 099 497        US-B1- 6 338 723**

**Description**

**Technical field of the invention**

The present invention relates to an elastic bandage useable e.g. for applying a controlled compression to parts of the body, the reason for said use being at least one of prophylactic treatment, therapeutic treatment, performance-enhancing and recovery. The invention is also related to an elastic material useable for such use, i.e. at least one of prophylactic treatment, therapheutic treatment, performance-enhancing and recovery.

**Background**

An elastic bandage (also known as elastic wrap, or compression bandage is a stretchable bandage used to create localized pressure. Elastic bandages are commonly used to inhibit or treat established swelling. Elastic bandages are also used to enhance the venous return in individuals with either venous insufficiency or obstruction. For non-medical indications elastic bandages are applied to reduce the risk of muscle sprain and strains and to improve recovery after physical activities.

Another medical application of bandage is to deliver a drug, for example through a dressing applied on the bandage.

A particular line of use for elastic bandages is for compression therapy designed for the purpose of venous edema. Such elastic bandages are specifically designed to enhance the transportation of blood from the superficial venous system into the deep venous system. Yet another purpose is to facilitate the transportation of blood in the deep system.In addition, compression is of value to prevent swelling to emerge or reduce risk for venous thrombus formation. Examples of the latter are in connection to surgery or transcontinental flights. Swelling can also commonly be seen in connection to heart failure.

Many medical conditions can be improved by compression therapy. The conditions to be improved include bleeding after surgery or bleeding following trauma. The bleedings following surgery or trauma can be located within different compartments or combination of compartments. These are characterized to be at different levels, depth in relation to the skin surface. Illustrative examples are subcutaneous bleedings following operations for varicose veins, muscular bleeding following orthopedic interventions or blunt trauma in contact sports with bleeding either subcutaneously or in muscles or within both locations in combination. Depending on localisation of the bleeding, different pressures need to be applied.

In sports, elastic compression bandages and garments are today being used in order to: improve recovery, faster warm up and enhance overall circulation, enhance stability and agility, reduce fatigue and reduce damage.

All indications and problems discussed above are also applicable in different settings in veterinary medicine.

The variety of indications needs a variety of different pressures to be applied for optimal function. By varying the ratio of e.g. cotton, polyester, and the elastic yarns within an elastic bandage, combined with different production methods various grades of compression and durability may be obtained in such elastic bandages.

Depending on indication (therapy, prevention) and targeted location, the needed pressure to be applied varies. However, applying an elastic bandage in an appropriate way with an appropriate pressure, and especially for compression therapy, requires great skill and experience, and there is also a great risk that bandages are applied in a non-optimal way. Since, the needed pressure varies with the indication and condition, the optimal application is difficult to reach and apply by hand. It is obvious that an elastic bandage, which can be applied by medical and non-medical individuals at precisely the pressure magnitude wanted, would solve very common and general medical problems.

There is therefore a need for an improved elastic bandage to alleviate the above-discussed problems.

Elastic pressure bandages are frequently, but not exclusively, applied to legs. However, a leg varies greatly in thickness along its length, and also varies greatly among different individuals, which makes application even more problematic. For application on legs, it can sometimes be desirable to let the applied pressure decrease in the proximal direction. As legs typically have a tapered shape, having greater circumference in the proximal direction, a pressure that decreases in the proximal direction can be achieved by letting the force, the width, and the overlap all be constant. This is a common approach in the art, as disclosed e.g. in WO2008/152294, US5779659, US5749843, US361 3679 and US2009/099497.

From these documents, it is e.g. known to provide visual indications, such as printed rectangles on the bandage, which changes in geometry as the elastic bandage is stretched, thereby indicating that the elastic bandage is stretched to a specific force. However, controlling the force properly is still extremely difficult. For example, the pressure strongly depends on the leg circumference, whereby the visual indications are only providing adequate pressure for a certain leg size. Even then, not all legs have the same tapered shape, and hence the desired graduated pressure may still not be achieved. GB 2104558 and US 6050967 disclose examples of knitted elastic bandages.

Further, WO98/47452 discloses an elastic bandage that when stretched by a specific force yields a graduated pressure when applied on a uniform cylindrical shape. In one embodiment, this is achieved by printing a guideline on the elastic bandage. When the elastic bandage is wrapped around the body part along this guideline, the overlap - and hence also the pressure - either increases or decreases. In another embodiment, the width of the elastic bandage changes in the longitudinal direction. Another method, disclosed in US5195950, of achieving a graduated pressure is to have markings on the elastic bandage that indicate the desired elongation, but then vary the markings in the longitudinal direction to require less or more elongation. In all cases it is necessary to start bandaging at either the distal or the proximal end of the limb, where the choice depends on the elastic bandage. Furthermore, the applied pressure on a body part with strongly tapered shape might get too large, and different leg sizes require different symbols.

A different approach to controlling the pressure is to make use of the fact that the elastic bandage will meet itself as it is wrapped around a body part. This approach is also known in the art. For instance, US6338723 discloses a therapeutic garment comprising a band with scales and reference lines that is wrapped around a body part. As the stretched distance between the scale and the reference line must coincide with the circumference of the body part - which has been measured in advance - the elongation of the band is known. Hence, the force, the width and the radius of curvature are all known, and the corresponding pressure can be printed as a scale on the garment, with different scales for different circumferences. The circumference itself can be measured by first applying the band unstretched; in essence using it like an ordinary tape measure. A similar elastic bandage is disclosed in WO03/005945 and the idea of wrapping a measurement strip around a body part to determine a suitable bandage or garment is disclosed in US6415525. However, also with these elastic bandages, it is difficult to obtain a suitable application for different sizes and shapes of legs.

Thus, there is a need for an elastic bandage, and in particular an elastic compression bandage, that yields a consistent pressure on a body part without requiring any measurements of circumference, and which can be applied to limbs of varying size and shape. Furthermore, the same elastic bandage will in principle be able to be applied in situations where it is desirable to let the pressure decrease in the proximal direction.

## Summary of the invention

There is therefore an object of the present invention to provide an elastic bandage and a textile material useable for such use that at least partly overcome the above-discussed problems of the prior art.

This object is achieved by means of an elastic bandage according to the enclosed claims.

According to a first aspect of the invention there is provided an elastic bandage comprising an elongate strip of a stretchable and elastic textile material, wherein a first set of repeated markings are provided and distributed along the longitudinal direction of the elongate strip for correlation of markings within different turns, allowing turns having the same length of unstretched elastic bandage to be wrapped and stretched around various circumferences with different yield rates, and wherein the elastic property of the elongate strip is such that the pressure exerted by such turns having the same length of unstretched elastic bandage varies less than 30% over a range of approximately circular circumferences providing a range of yield rates from $\lambda_1$ to $\lambda_2$, wherein $\lambda_2/\lambda_1 > 1.8$.

The present invention is based on the realization that when an elastic bandage is stretched and wrapped around a body part, the body part will be compressed by a normal force. As the force locally is close to constant, it is convenient to instead consider the pressure, the force per surface area. Theoretically, this pressure is given by the longitudinal force in the elastic bandage, divided by the product of the stretched elastic bandage width and the local radius of curvature. For body parts that can be considered to have circular cross sections, such as limbs, the radius of curvature can be approximated by the radius of a circle with the same circumference as the body part.

As the elastic bandage is usually wrapped around the body part with a certain overlap between the turns, the total pressure is larger than that from a single turn. If each turn advances a fraction of the elastic bandage width given by a constant inverse integer - 1/1, 1/2, 1/3, 1/4, ... - then the total pressure is, in theory, the pressure from a single turn multiplied by the corresponding integer - 1, 2, 3, 4, ... - since there are that many bandage layers overlying each part of the area. For a general fraction, the number of bandage layers will not be constant. However, the average number of layers is given by the linear interpolation between the neighbouring integers, as a function of the fractional shift. In real life applications, the thickness of the elastic bandage is also much smaller than the radius of curvature. At the very beginning and end of the elastic bandage the number of turns, and hence the pressure, may be lower, unless the degree of overlap is changed there, which however, is a common practice.

Hence, in order to achieve the desired overall pressure it is important to control the longitudinal force in an elastic bandage, the width of the bandage, the overlap between the turns, as well as knowing the radii of curvature. However, whereas the overlap and width are easy to determine when applying an elastic bandage, the longitudinal force is far more complex to predict. Further, the combination of these variables makes proper real-life application of elastic bandages a very demanding task. Improper and non-optimized application of bandages may lead to an inferior prophylactic and therapeutic results, and in worst cases even to pain and serious side-effects for the patient.

The elastic bandage of the present invention is based on the realization that use of an elastic bandage with markings allowing each turn to use the same amount of unstretched length of the elastic bandage, and at the same time use of a stretchable elastic textile material having an elastic property such that the pressure exerted by such turns having the same length of unstretched bandage varies less than 30% over a range of approximately circular circumferences providing a range of yield rates from $\lambda_1$ to $\lambda_2$, wherein $\lambda_2/\lambda_1 > 1.8$ is advantageous. Hereby, application can be made accurate, and at the same time providing a very uniform and predictable pressure. The same elastic bandage may also be used on different patients, different body parts, etc., since the same result will automatically be achieved regardless of the size of the circumference - and hence the radius of curvature, over a large range of diameters.

The engineering principles of the present invention are as follows. If we consider a stretched elastic bandage wrapped around the axis of symmetry of the frustum of a right circular cone with radii ranging from $r_1$ to $r_2$, where $r_1 < r_2$, then the pressure on the cone is given by the formula

$$p = \frac{F\,n}{w\,r},$$

where $F$ is the longitudinal force in the elastic bandage; $n$ is the number of layers; $w$ is the width of the elastic bandage; and $r$ is the radius. In the

medical literature, this formula is sometimes referred to as Laplace's law. This formula assumes that the longitudinal stress in the elastic bandage - which is easy to measure - coincides with the hoop stress. However, if the elastic bandage progresses along the axis of the cone as it is wrapped around the cone, then these quantities no longer coincide. Nevertheless, the difference is quite small when the overlap is 50 percent or more, and it can usually be accounted for by calibration. Furthermore, in this case the number of layers $n$ should be interpreted as the average number of layers, where the boundary effects are ignored. That is, if the elastic bandage at every turn is shifted a fraction $t$ of the bandage width, then $n$ is the linear interpolation, as a function of $t$, between $1/t$ rounded down to the nearest integer and $1/t$ rounded up to the nearest integer. The closer $1/t$ is to an integer, the closer to constant the average number of layers will be.

Now we may introduce the stretch quotient, also referred to as the yield rate, $\lambda$, defined by

$$\lambda = \frac{L}{L_0}$$

where $L, L_0$ are the stretched and unstretched lengths, respectively.

Further, the stretched length will be $L = 2\pi r$, or equivalently $r = \lambda \frac{L_0}{2\pi}$, and from this we see that

$$p = \frac{2\pi F\,n}{w\,\lambda\,L_0} \qquad\qquad (1)$$

Now, let $p_0$ denote the desired lowest pressure and, by way of example only, assume that the desired smallest overlap is 50 percent, so that $n = 2$. Then as long as

$$\frac{F}{w\,\lambda} = \frac{p_0 L_0}{4\pi} \qquad\qquad (2)$$

the applied pressure is $p_0$ when the elastic bandage is wrapped with an overlap of 50 percent. By changing the overlap, and hence n, the applied pressure changes by a factor of $n/2$.

With the elastic bandage material of the present invention, this relation (2) approximately holds for stretch quotients covering all relevant radii. That is, it approximately holds at least for $\lambda$ satisfying

$$\frac{2\pi r_1}{L_0} \leq \lambda \leq \frac{2\pi r_2}{L_0}. \qquad\qquad (3)$$

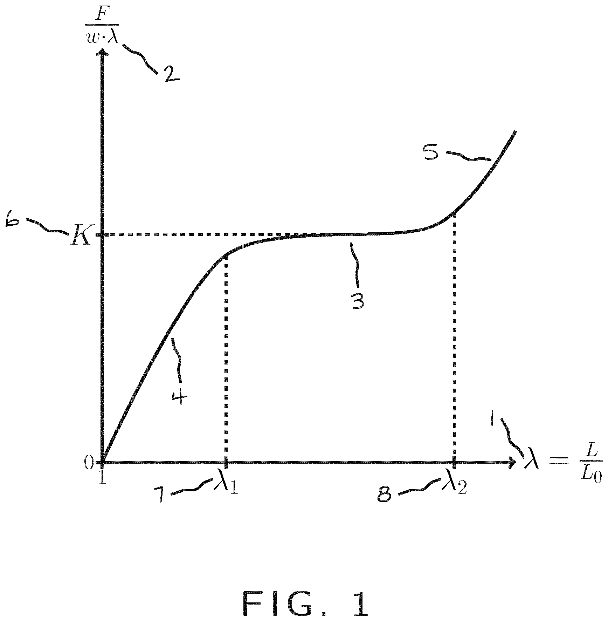

In practice, $\frac{F}{w\,\lambda}$ is measured experimentally and is found to be approximately a constant K for $\lambda$ in some interval $\lambda_1 \leq \lambda \leq \lambda_2$, see FIG. 1. In this case, we may choose the unstretched length $L_0$ as

$$L_0 = \frac{4\pi K}{p_0},$$

and the pressure $p_0$ then satisfies

$$\frac{2\,\lambda_1 K}{r_1} \le p_0 \le \frac{2\,\lambda_2 K}{r_2}$$

In particular, one single elastic bandage type may be used for multiple target pressures by varying the length $L_0$. Furthermore, we see from (1) that if the left hand of (2) is constant up to a certain relative error, then the pressure is constant up to the same relative error, when $n$, $w$, and $L_0$ are fixed.

As the elastic bandage of the present invention satisfies equation (2) with only a small error, the present invention offers an elastic bandage that yields a homogeneous pressure on body parts of varying circumference.

It may also easily be made sure that the resulting elastic bandage is not used outside the domain given in (3). For example, it is sufficient to measure - or often to just estimate the size of - the body part where it is the narrowest and also where it is the widest. In the case of legs, this is usually at the ankle and at the upper thigh, respectively, and in all cases it suffices to measure the circumference of the body part at two points only.

That the elongation is within the desired domain can also be verified using any of the techniques known in the art for indicating elongation. For instance, one may print two sets of rectangles on the elastic bandage with side lengths chosen so that they become squares at elongations $\lambda_1$ and $\lambda_2$, respectively. In this latter case, it is not necessary to do any measurements of circumference. Furthermore, these elongation indicators may be incorporated in the repeated transversal markings used by the present invention.

With the present invention, every turn of the elastic bandage preferably makes use of the same amount of bandage material, determined by the unstretched length $L_0$ measured in the longitudinal direction of the bandage. Furthermore, every turn of the elastic bandage progresses a certain length - determined solely by the bandage width and the overlap - in the longitudinal direction of the body part.

Hence, if the elastic bandage used is correlated to the length of a body part and wrapped around it by starting at specified ends of both the body part and the elastic bandage, then it is possible to determine exactly what part of the elastic bandage that will cover any specified longitudinal section of the body part. By varying the elastic bandage in the longitudinal direction - for instance by making changes in the distance between the marks, or by making the elastic bandage thicker or otherwise changing the elastic bandage material - it is then possible to also vary the pressure in a predictable and predetermined way.

By way of example, consider the bandaging of legs. If the person is standing up, then gravity will cause the pressure in the blood vessels to increase in the distal direction. According to physiological studies, the increase of pressure at a point is approximately proportional to the height of the blood column between the point and the heart. In a healthy blood vessel, this increase in pressure is countered by a corresponding increase in pressure in the surrounding tissue, since the densities are almost the same. However, in case of certain medical conditions, such as deep venous insufficiency, an additional pressure has to be applied at all times, and to achieve this, the forces of gravity might need to be countered by applying the bandage with an decreasing pressure in the proximal direction.

As the present invention makes it possible to vary the pressure in the longitudinal direction of the body part, an elastic bandage can be constructed that automatically yields a decreasing pressure in the proximal direction. Note that one single elastic bandage made out of a textile material satisfying equation (2) will yield the same graduated pressure for varying shapes of the legs.

In a similar manner, other types of graduated pressure - including arbitrary pressure changes in the longitudinal direction of the body part - can also be achieved.

Some patients in compression therapy require an individually customized pressure. For instance, the pressure should be lower over certain wounds or sites with sensitive skin. Traditionally, this is achieved by using pads that either increase or decrease the pressure. However, this method is imprecise and difficult to apply. As the present invention makes it possible to create elastic bandages with graduated pressure, it is also possible to create elastic bandages with an individually customized pressure profile. To manufacture such a customized bandage from a textile material satisfying equation (2), one only needs to know the desired pressure at different longitudinal sections of the body part, which is very easy to specify. In particular, it is not necessary to measure the circumference of the body part.

For certain medical conditions, it is desired that the applied pressure on the leg is higher when standing up than when lying down. Customarily, this is achieved by making use of the fact that fluid accumulates in the legs, thereby increasing their volume, when standing up. If the elastic bandage is such that the force in the elastic bandage increases rapidly with the elongation, then the increase in volume causes an increase in pressure.

However, the changes in volume are quite small. This means that the elastic bandage has to be stretched a very precise amount in the longitudinal direction when applied, otherwise the pressure on the body part can get far too large.

With an elastic bandage of the present invention, which preferably satisfies equation (2), then there will be no increase at all in the pressure from the elastic bandage when the person stands up. For some situations, such as for certain patients who are mainly lying down, this is desirable. If, however, an increase in pressure is desired, this can still be achieved using the present invention: e.g. by applying a second bandage - where the order of application does not matter - essentially unstretched but of a 'stiff' fabric, so that a small increase in elongation causes a large increase in the force. As this second non- elastic bandage is to be applied at close to zero force/pressure, one avoids the drawbacks of previous solutions. The second non- elastic bandage could also be fastened in the first, for instance by using a hook-and-loop fastener.

Preferably, the elastic properties of the textile material are such that the pressure exerted by such turns having the same length of unstretched elastic bandage varies less than 20% over a range of circumferences providing a range of yield rates from $\lambda 1$ to $\lambda 2$, and even more preferably varies less than 10%. Further, it is preferred that the range of yield rates in which this property exist are such that $\lambda 2/\lambda 1 > 1.9$, and most preferably $\lambda 2/\lambda 1 > 2.0$. Hereby, an even more flexible and useful elastic bandage is obtained. In particular it is preferred that the elastic properties of the textile material are such that the pressure exerted by such turns having the same length of unstretched bandage varies less than 20% over a range of circumferences providing a range of yield rates from $\lambda 1$ to $\lambda 2$, wherein the range of yield rates in which this property exist are such that $\lambda 2/\lambda 1 > 1.9$, Even more preferred, the elastic properties of the textile material are such that the pressure exerted by such turns having the same length of unstretched elastic bandage varies less than 10% over a range of circumferences providing a range of yield rates from $\lambda 1$ to $\lambda 2$, wherein the range of yield rates in which this property exist are such that $\lambda 2/\lambda 1 > 2.0$, Hereby, an even more flexible and useful elastic bandage is obtained. The markings are preferably visually and/or tactilely discernible. For example, the markings may be printed markings, discernible structures in the textile material, openings, etc.

Markings may also be provided to determine the degree of overlap between different turns. Hereby, a desired degree of overlap may easily be obtained during application. This allows the overlap to be constant at all places. However, alternatively, the markings may enable an overlap which is varied in a predicted and predetermined way. In one embodiment, the markings indicating the desired overlap may be the same first set of markings, which are also used to determine the length of elastic bandage material to be used for each turn. However, alternatively a second set of markings is provided to determine the degree of overlap between different turns. In such an embodiment, the second set of markings may comprise markings arranged along at least one line extending in the length direction of the elastic bandage.

The first set of markings preferably comprises marking lines extending at least partly in a transversal direction of the elastic bandage.

The first set of markings preferably comprises markings extending over more than 50% of the width of the elastic bandage. Hereby, the markings are clearly visible, even when a substantial overlap has been applied. However, alternatively the first set of markings may comprise markings having a limited extension in the width direction of the elastic bandage, and being arranged in the vicinity of at least one of the longitudinal sides of the elastic bandage.

The first set of markings may e.g. comprise characters or digits forming a continuous or discontinuous scale in the length direction of the elastic bandage. This may also enable the use of different sets of markings for correlation between the turns, thereby making it possible to apply the elastic bandage with different pressures.

The first set of markings preferably comprises at least one set of markings which are repeatedly and equidistantly arranged in the lengthwise direction of the elastic bandage.

The elastic bandage material is preferably a textile, and preferably comprising both elastic and non-elastic yarns and threads.

The textile material preferably comprises synthetic fibres selected from the group consisting of polyester, polyamide, polypropylene or PLA (polylactic acid). In addition, the textile material may further comprise natural fibres, such as cotton or regenerated fibres such as viscose or a mixed spun yarn with multifilament synthetic fibres and natural staple fibres or other mixtures thereof. The textile material preferably comprises threads or yarns of at least one elastic material, said elastic material comprising at least one of: elastomeric polymers such as natural rubber (i.e. polyisoprene), synthetic rubber, a mix of polyisoprene rubber and styrene butadiene copolymer or a mix of thermoplastic and elastomeric polymers such as polyurethane-polyurea copolymer, and other mixtures thereof.

It has been found that these elastic materials are very useable for obtaining the above-discussed elastic properties.

According to another aspect there is provided a use of a textile material in the manufacture of an elastic bandage, the textile material having the elastic property that when the same length of unstretched elastic bandage is stretched in turns over a circular object with different yield rates the pressure exerted by such turns having the same length of unstretched elastic bandage varies less than 30% over a range of approximately circular circumferences providing a range of yield rates from $\lambda_1$ to $\lambda_2$, wherein $\lambda_2/\lambda_1 > 1.8$.

Hereby, similar advantages and specific features as discussed above in relation to the first aspect are obtainable and useable.

According to still another aspect there is provided a stretchable elastic bandage material for obtaining compression having the elastic property that when the same length of unstretched elastic bandage material is stretched to encircle circular object with different circumferences at different yield rates the pressure exerted by the elastic bandage varies less than 30% over a range of approximately circular circumferences providing a range of yield rates from $\lambda_1$ to $\lambda_2$, wherein $\lambda_2/\lambda_1 > 1.8$.

Hereby, similar advantages and specific features as discussed above in relation to the first aspect are obtainable and useable.

Such a textile material may also be used for other types of prophylactic and therapeutic compression hosiery applications, such as in tubular bandages, compression stockings, compression socks and the like.

The above-discussed elastic bandage and textile material are particularly useful for applying a controlled compression to parts of the body, for use in at least one of prophylactic treatment, therapeutic treatment, performance-enhancing and recovery. However, in addition to compression therapy, it is also useful for other known uses of elastic bandages. For example, it is useful for the above mentioned drug delivery through dressing coated bandage, where the drug will be distributed according to the area of the bandage on the skin. It is a strength that this area, of unstretched bandage material, can be independent of the radii of the limb, thus making sure that a well-defined dosage of the said drug is delivered. Hence the present invention is highly suited for such application.

The elastic bandage of the present invention is useable to create localized pressure, e.g. to inhibit or treat established swelling. The elastic bandage may also be used to enhance the venous return in individuals with either venous insufficiency or obstruction. The elastic bandage is also useable for non-medical indications to reduce the risk of muscle sprain and strains and to improve recovery after physical activities.

A particular line of use for the elastic bandages is for compression therapy designed for the purpose of venous edema. Yet another use is to facilitate the transportation of blood in the deep system. A subgroup of elastic bandages with this purpose deliver graduated compression from the ankle and in proximal (towards the heart) direction.

The elastic bandage is also useable to prevent swelling to emerge or reduce risk for venous thrombus formation. Examples of the latter are in connection to surgery or transcontinental flights. Swelling can also commonly be seen in connection to heart failure.

The elastic bandage is also useful as sport equipment, in order to: improve recovery, faster warm up and enhance overall circulation, enhance stability and agility, reduce fatigue and reduce damage.

Further, even though the elastic bandage and textile material are intended for use on humans, it is equally possible to use the elastic bandage and textile material for usage on animals.

These and other aspects of the invention will be apparent from and elucidated with reference to the embodiments described hereinafter.

**Brief description of the drawings**

For exemplifying purposes, the invention will be described in closer detail in the following with reference to embodiments thereof illustrated in the attached drawings, wherein:

Fig. 1 schematically illustrates the elastic and mechanical property of an elastic bandage material in accordance with an embodiment of the invention.
Fig. 2 illustrates the elastic and mechanical properties for some exemplary elastic bandage materials in accordance with the invention, and, as a comparison, for some previously used elastic bandage materials.
Figs. 3-20 show an elastic bandage with markings in accordance with different embodiments of the present invention.
Fig. 21 shows an exemplary embodiment of a textile material structure useful for an elastic bandage material in accordance with the present invention.

**Detailed description of preferred embodiments**

In the following detailed description, preferred embodiments of the present invention will be described. However, it is to be understood that features of the different embodiments are exchangeable between the embodiments and may be combined in different ways, unless anything else is specifically indicated. It may also be noted that, for the sake of clarity, the dimensions of certain components illustrated in the drawings may differ from the corresponding dimensions in real-life implementations. Even though in the following description, numerous specific details are set forth to provide a more thorough understanding of the present invention, it will be apparent to one skilled in the art that the present invention may be practiced without these specific details. In other instances, well known constructions or functions are not described in detail, so as not to obscure the present invention. Further, the same reference signs are used to designate equal or similar parts throughout the drawings.

In Fig. 1, the elastic and mechanical properties of the elastic bandage material in accordance with an embodiment of the present invention are illustrated schematically. The diagram shows the longitudinal force divided by the product of the stretched elastic bandage material width and the stretch ratio 2 as a function of the stretch ratio 1. The stretch ratio 1 is defined as the stretched ratio of the elastic bandage divided by the unstretched length. An optimal elastic textile material has a range 3, determined by two stretch ratios 7, 8, where the quantity 2 is close to a constant 6. As has been discussed in the foregoing, slight deviations from such a constant can be tolerated, and still provide a highly useable elastic bandage material. The quotient of 8 and 7 determines the possible variation in circumference of the body parts - including how tapered they may be - where the elastic bandage is applied, and it should preferably exceed 1.8, more preferably exceed 1.9, and most preferably exceed 2.0. How close the quantity 2 is to a constant 6 when the elongation is between 7 and 8 determines the precision of the pressure applied by the bandage, and preferably 2 varies less than 30 percent from 6 in the interval, more preferably less than 20 percent, and most preferably less than 10 percent. The behaviour 4, 5 outside of the interval determined by 7 and 8 is of less interest.

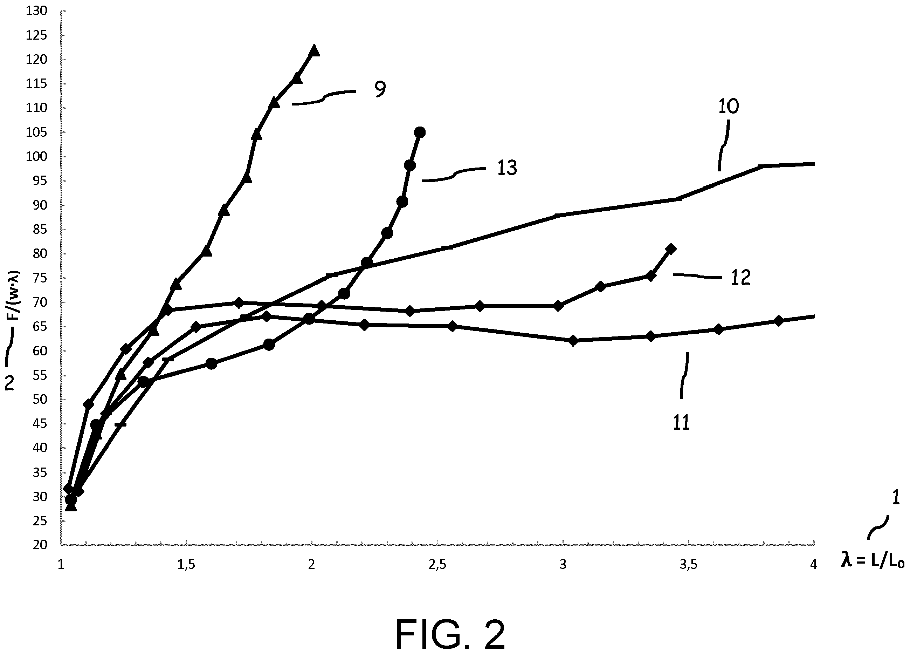

Fig. 2 shows experimentally determined values of the quantity in Fig. 1 for the commercially available Dauerbinde K 9 (produced by Lohmann & Rauscher), Refit Band XX-Light 10 (made from natural latex rubber and sold by Mediband AB, Nacka, Sweden), Synthetic rubber threads 2L39 11 (produced by Fulflex Elastomeric Worldwide, USA), and SuperElastic 13 (of 10 cm width, sold by Invivo Trade AB, Ängelhom, Sweden under the brand Masita Sports Medical). As 12 an elastic bandage corresponding to the present invention is illustrated. The textile material of the elastic bandage 12 in this case comprises synthetic rubber threads 2L39.

Notably, only items 9, 12 and 13, i.e. the Dauerbinde K, the bandage of the present invention and the SuperElastic are bandages. The Refit Band XX-Light 10 is a homogeneous natural latex rubber tape, which is not useable as a bandage. Instead, such tapes are used for rehabilitation and training exercises. This material is included only as a comparative example. Further, the synthetic rubber threads 2L39 are threads, not per se useable as bandages. Instead, these threads are useable e.g. for manufacturing clothes and technical textiles.

In a further experiment, two conventional bandages and a bandage in accordance with the invention were applied on a model of a human leg. The bandages used were the same bandages as discussed above in relation to Fig. 1, viz. Dauerbinde K, SuperElastic and a bandage in accordance with the present invention. In the following, the Dauerbinde K is referred to as CEA (Comparative Example A), the SuperElastic as CEB (Comparative Example B), and the bandage of the present invention as IE (Inventive Example).

The bandages were applied in accordance with the manufacturer's instructions. The Dauerbinde K and the SuperElastic do not have any markings or the like to guide during application. Instead, the overlap and stretching are continuously controlled and guided by the experience of the bandage applier. The bandage in accordance with the present invention comprised longitudinally displaced markings at 21 cm marking distance, and was applied with 3/5 overlap. The bandages were made applied by two independent bandage appliers, each having sufficient medical training and experience of applying bandages on patients.

The pressure in various parts of the leg was determined by PicoPress pressure sensors. Three PicoPress sensors were placed at the back (dorsal) of the leg. The first was placed at a thigh position, at a circumference of 47 cm. The second was placed at the calf, at a circumference of 35.5 cm. The third was placed at the wrist, positioned 8 cm above lateral malleolus, at a circumference of 24 cm. The measured pressure at the various parts are presented in the following table (the values being the pressure in mmHg):

|  | Bandage applier 1 | | | Bandage applier 2 | | |
|---|---|---|---|---|---|---|
|  | IE | CEA | CEB | IE | CEA | CEB |
| Thigh | 38 | 53 | 58 | 38 | 27 | 24 |
| Calf | 36 | 72 | 78 | 35 | 34 | 18 |
| Wrist | 31 | 55 | 55 | 32 | 44 | 30 |
| Mean | 35 | 60 | 64 | 35 | 35 | 24 |
| Variance | 13 | 109 | 156 | 9 | 73 | 36 |

Thus, this experiment shows that by using a bandage in accordance with the present invention, the pressure obtained at various leg parts, having various diameters, is essentially uniform, whereas in the conventional bandages used as comparative examples, the pressure varies very significantly. The result obtained by means of the bandage in accordance with the invention is also highly predictable - almost identical results were obtained by the two independent bandage appliers - whereas the results differed significantly between the two bandage appliers when using the bandages of the comparative examples. Notably, combining the results of the two bandage appliers, the total variance of the inventive example is 9, compared to 260 for comparative example A (the Dauerbinde) and 549 for comparative example B (the SuperElastic).

Thus, it has been shown that bandages according to the present invention provide much lower pressure variations in different parts of the leg, and thus are more insensitive to variations in circumference, and also provide improved predictability and controllability, compared to bandages of the prior art.

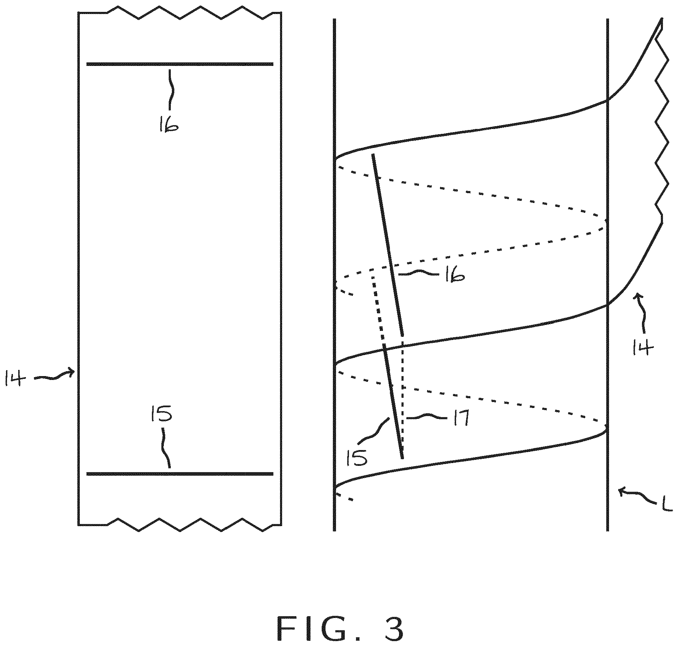

Fig. 3 shows one embodiment of the elastic compression bandage 14, when unstretched (to the left) and when wrapped around a body part L in a stretched state (to the right). Repeated markings 15 and 16 are here provided as lines extending transversely to the longitudinal direction of the elastic bandage. The lines extend over the whole, or almost the whole width of the elastic bandage. At every turn except the first, the elastic bandage is stretched so that the transversal markings 15 and 16 - from the previous and current turn, respectively - become translates of each other in the longitudinal direction 17 of the body part L. In another embodiment, the transversal markings could instead align.

The transversal markings could be printed on both sides or on one side of the elastic bandage 14, and the markings need not be the same on both sides. The transversal markings could also be embedded into the textile material of the elastic bandage. In one embodiment markings are printed on the skin-side of the bandage 14 and the elastic bandage is at least partly transparent when stretched. In another embodiment markings are printed on the skin-side of a bandage 14 which is opaque when stretched, but still serve a purpose as they are visible when the elastic bandage is applied.

In yet another embodiment the markings are invisible to the human eye and have to be read by different means, such as tactilely, electrically, by x-ray, under ultraviolet light, or magnetically. In still another embodiment the markings are present when the elastic bandage is applied, but fade away over time, for instance due to withering, a chemical reaction, or for some other reason initiated by the stretching of the elastic bandage.

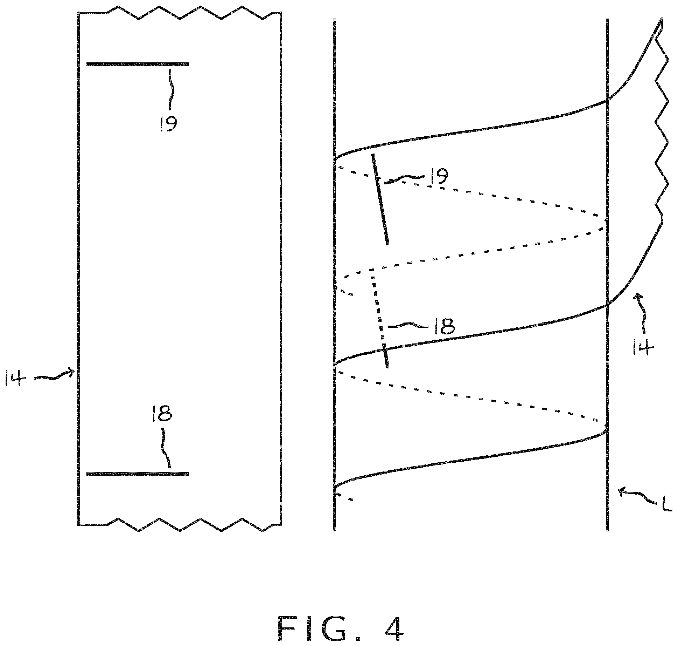

Fig. 4 shows an embodiment - similar to that in Fig. 3 - where the transversal markings 18 and 19 only extend partly across the elastic bandage 14. In this embodiment it is easier to estimate the overlap between different turns.

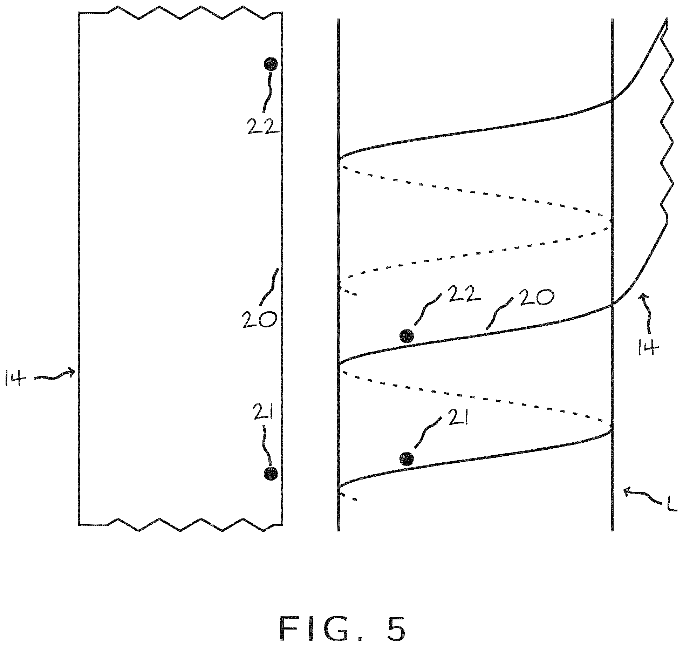

Fig. 5 shows an embodiment - similar to that in FIG. 3 - where the transversal markings are reduced to marks 21 and 22 near the edge 20 of the elastic bandage 14.

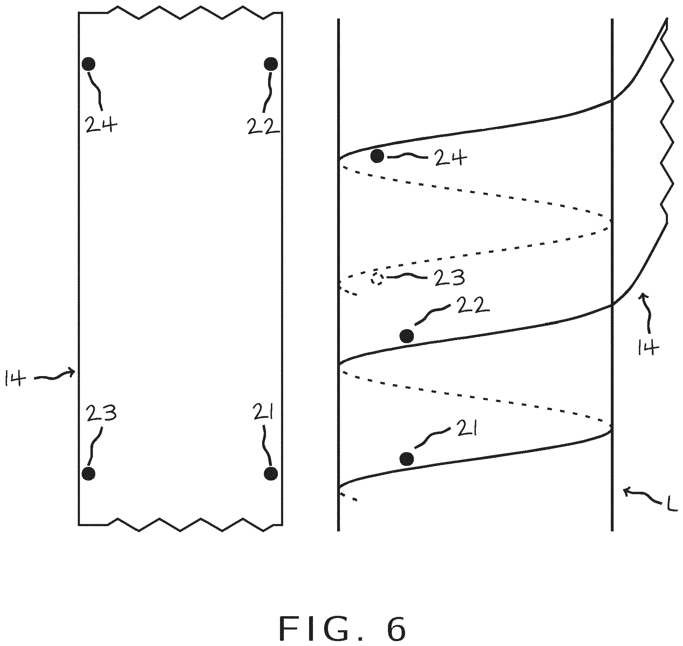

Fig. 6 shows an embodiment similar to that in Fig. 5, but having also transversal markings 23 and 24 near the opposite edge. In this embodiment the elastic bandage can be wrapped in any direction around the body part L without hiding the markings.

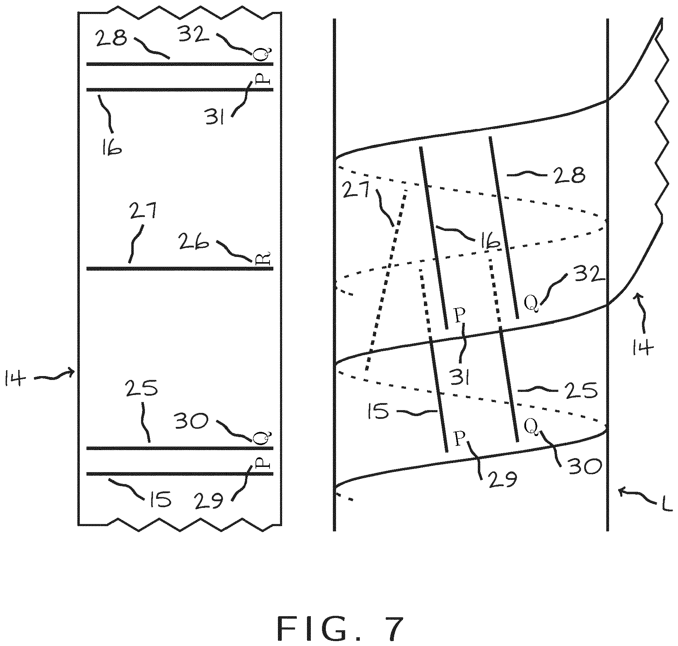

Fig. 7 shows an embodiment where there are several transversal markings 15, 25, 27 (first turn shown), 16, 28 (second turn shown) for every turn. As the elastic bandage 14 is wrapped around the body part, the pairs 15, 16 and 25, 28 are matched as in Fig. 3. Labels 26, 29, 30, 31, 32 aid in finding the corresponding transversal markings. Having several transversal markings for every turn makes it easier to apply the elastic bandage with a consistent force during the turns.

In the embodiment in Fig. 7 there are three transversal markings for every turn, and the longitudinal distance between the two adjacent markings 15 and 25 is shorter than the distance between 25 and 27. In other embodiments there could be any number of transversal markings for every turn, and the distances between any pair of adjacent transversal markings may all be different. In another embodiment the markings 26, 29, 30, 31, 32 may be omitted. Yet another embodiment could instead of labels, or as a complement to labels, use transversal markings that are visually distinct, such as being of different colour or of varying stroke width, or comprising different symbols.

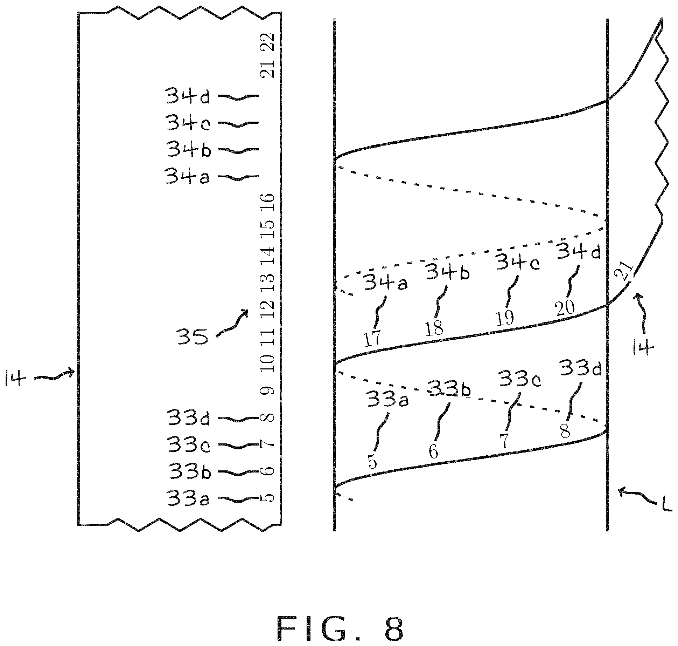

In the embodiment shown in Fig. 8 a scale 35 is printed in the longitudinal direction of the elastic bandage 14. The elastic bandage is then wrapped around the body part L in such a way that the difference between the scale elements 33, 34, matched as in Fig. 3, is given by a constant. Different constants correspond to different pressures, and the scale can be normalized so that the inverse of the constant yields the applied pressure in a customary pressure unit such as Pascal (Pa) or millimetre of mercury (mmHg). In one embodiment the scale 35 is monotonically and equidistantly increasing throughout the elastic bandage 14. In another embodiment the scale repeats itself at some interval longer than a desired shortest possible difference, and the difference between two scale elements 33, 34 is calculated modulo the length of the scale. In yet another embodiment the scale splits after some interval with one branch repeating itself, whereas the other only continues to increase monotonically for some distance and then ends.

The markings of this embodiment allow the user to use different constants when applying the elastic bandage, thereby using different lengths of unstretched elastic bandage material for each turn. Hereby, the application of the elastic bandage can easily be used for application of different pressures on different patients, and it may also be used to apply different pressure at different sections and the like.

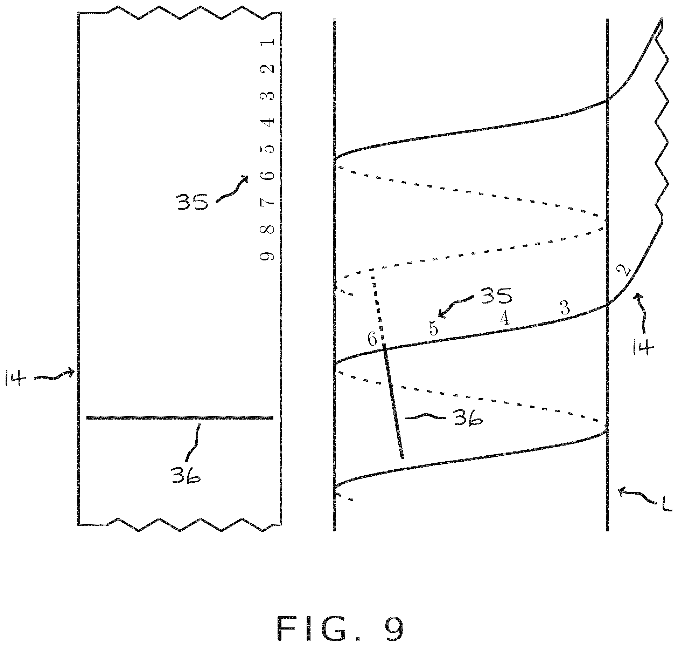

Fig. 9 shows an embodiment, similar to that in Fig. 8, where both transversal marking lines 36 and scales 35 are repeated in the longitudinal direction of the elastic bandage 14. As the elastic bandage is wrapped around the body part L at a specified overlap, the applied pressure can be read where the transversal marking line 36 intersect the scale 35, which may show the applied pressure in a customary unit such as Pascal (Pa) or millimeter of mercury (mmHg). In another embodiment, several scales may occupy the same longitudinal section of the elastic bandage.

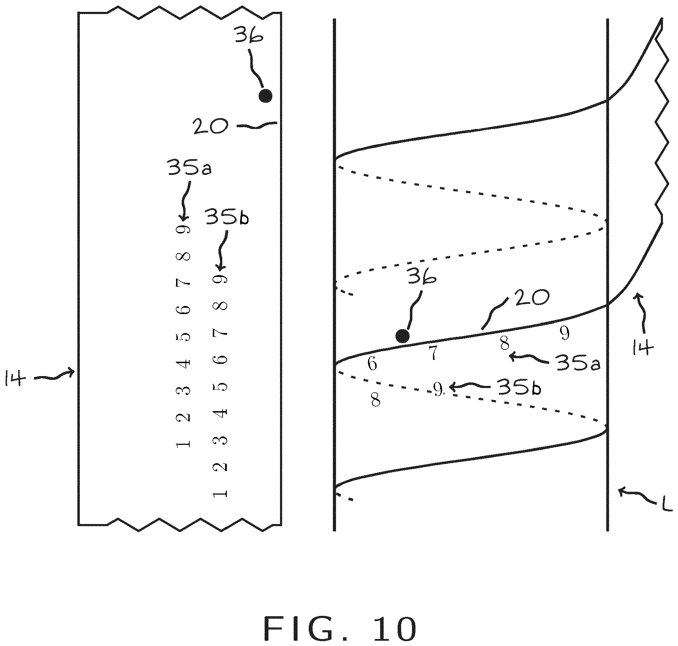

Fig. 10 shows an embodiment, similar to that in Fig. 9, but where the scale 35 and reference mark 36 have switched order. In this embodiment, the reference mark 36 can be reduced to a single marking near the edge 20 of the elastic bandage 14. Different overlaps are handled by including more than one scale 35a, 35b and reading the scale closest to the edge 20 that is not covered by the elastic bandage 14.

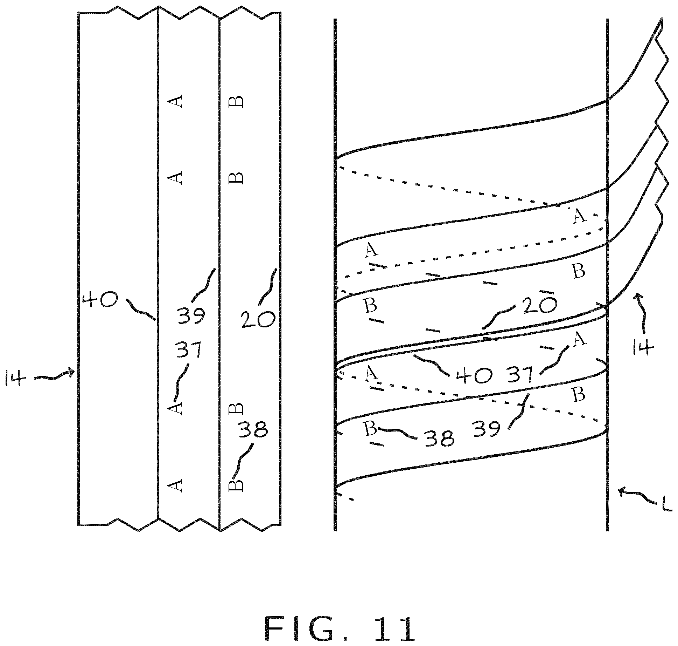

Fig. 11 shows an embodiment where the elastic bandage 14 is to be wrapped in a specified direction around the body part L, with the edge 20 aligning with a longitudinal marking 39, 40 depending on the desired overlap. The markings 37, 38 indicate the pressure resulting from that overlap, and also form transversal markings, similar to 15 and 16 in Fig. 3, that indicate the desired elongation of the elastic bandage. In other embodiments there could be any number of longitudinal markings 39, 40 and markings 37, 38.

It is also possible to use non-longitudinal lines, as is per se known from WO 98/47452.

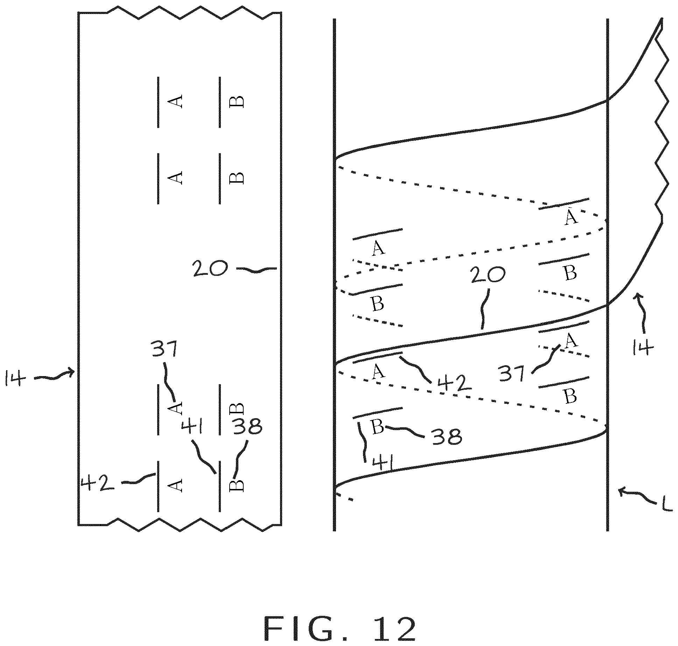

The embodiment in Fig. 12 is similar to that in Fig. 11, but has longitudinal markings 41, 42 that are present only at certain longitudinal sections of the elastic bandage. In another embodiment the labels 37, 38 are omitted, and instead the longitudinally confined markings 41, 42 indicate also the desired elongation; or vice versa in yet another embodiment. In other embodiments, those labels and markings 37, 38, 41, 42 that do not indicate the desired elongation may be longitudinally shifted or omitted.

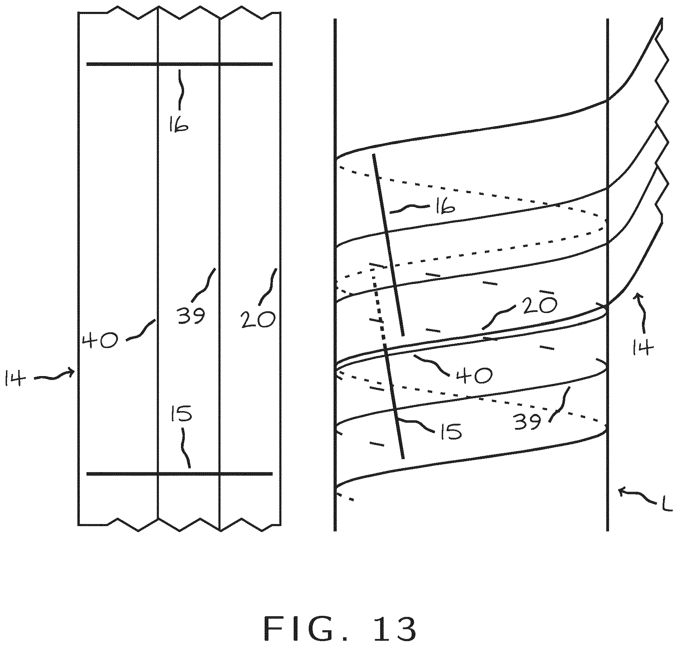

Fig. 13 shows an embodiment similar to that in Fig. 11, where the correct elongation is instead indicated by matching the transversal markings 15, 16 as in Fig. 3.

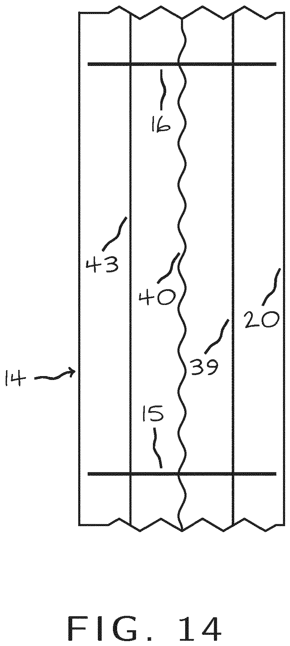

Fig. 14 shows an embodiment where one longitudinal marking 40 is transversally centred at the elastic bandage with the other longitudinal markings 39, 43 symmetrically distributed around it. In this case it is not necessary to wrap the elastic bandage in any specified direction around the body part.

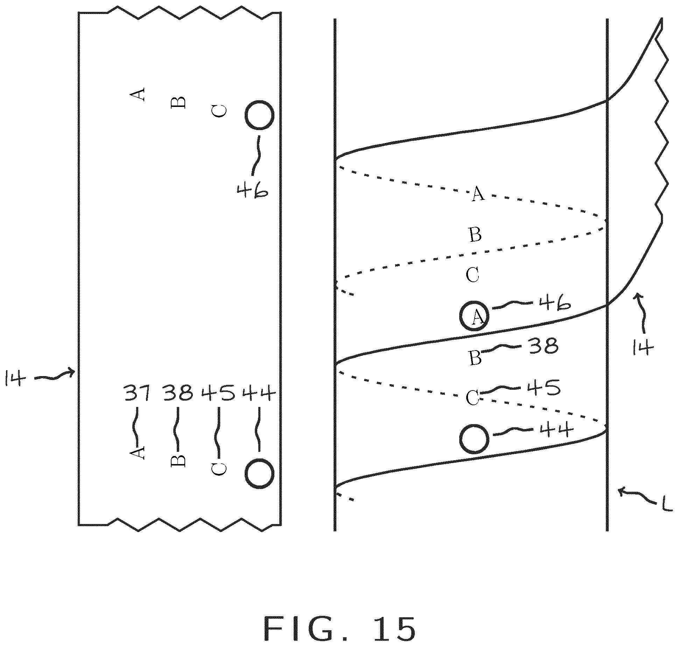

Fig. 15 shows an embodiment where the elastic bandage 14 has overlap markings 37, 38, 45, corresponding to different pressures, and holes/openings 44, 46. As the elastic bandage is wrapped around the leg L the markings 37, 38, 45 from the previous turn become visible through the hole 46 when the elastic bandage is wrapped with the correct elongation and overlap. In a similar embodiment the elastic bandage is sufficiently transparent to allow a reading through the fabric, and the hole 44, 46 is replaced by a marking on the elastic bandage itself. In another similar embodiment, the elastic bandage at large is not sufficiently transparent to allow a reading through the textile material, but the material used inside of the markings 44, 46 is. In all of the embodiments the markings 37, 38, 45 may be shifted in the longitudinal direction of the elastic bandage to facilitate the reading at the corresponding overlap.

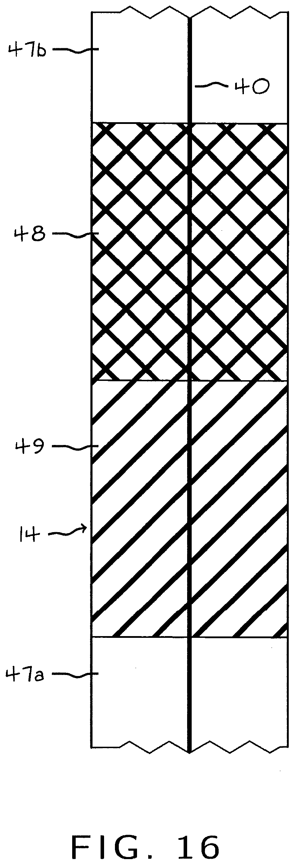

Fig. 16 shows an embodiment similar to Fig. 13 where the elastic bandage 14 consists of visually, tactilely, or otherwise distinct longitudinal sections 47, 48, 49. As the elastic bandage is wrapped around a body part, each turn consists of the same number of longitudinal sections, or alternatively varied in a consistent fashion. In another embodiment, the sections 47, 48, 49 have different longitudinal length. In yet another embodiment the sections are repeated in a regular fashion, and in still another embodiment the sections do not repeat in a regular fashion and are, for instance, instead permutated or all different. In all embodiments there are at least two, but otherwise any number of longitudinal sections.

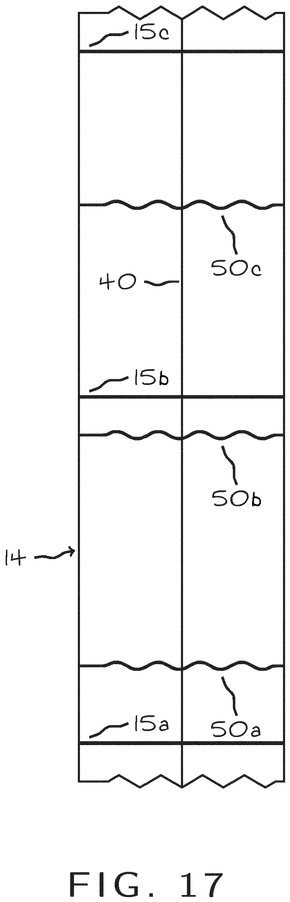

FIG. 17 shows an embodiment that is particularly suitable for different target pressures. The elastic bandage 14 has two sets of distinct markings 15 and 50. Wrapping the bandage 14 around a body part in such a way that 15a is matched with 15b and 15b with 15c - as in Fig. 3 - yields one specific pressure, and if instead 50a matches 50b which in turn matches 50c, then a different specific pressure is applied to the body part. In one embodiment, the distance between the marks within the different sets of indicators 15 and 50 is chosen as integer multiplies of each other, allowing transversal markings to be shared. In another embodiment, the markings of the different sets are instead shifted in the longitudinal direction with respect to each other in order to minimize the overlap between the sets.

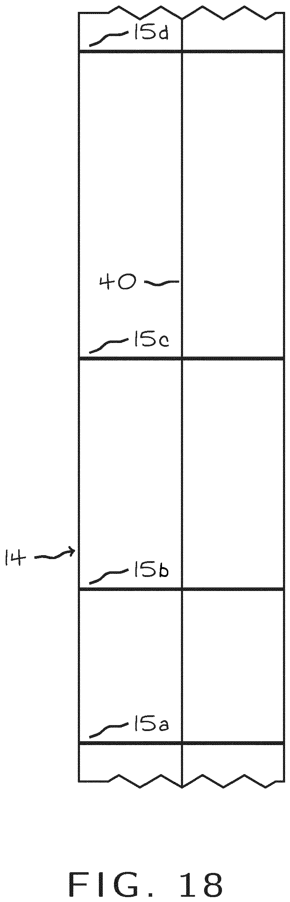

Fig. 18 shows an embodiment that yields a graduated pressure when wrapped around a body part with constant overlap. As in Fig. 3, the elastic bandage 14 is stretched so that the transversal markings 15 match, but in this embodiment the longitudinal distance between the transversal markings increase. For instance, the pressure applied by the bandage between 15a and 15b is higher than that between 15b and 15c, as the latter to markings are separated by a larger longitudinal distance.

Alternatively, the markings per se known from US 5195950 may be used.

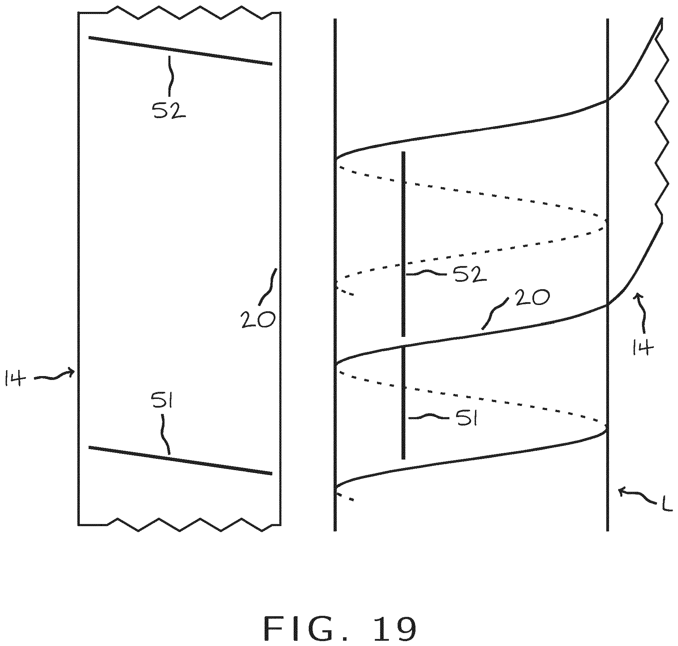

Fig. 19 shows an embodiment, similar to that in Fig. 3, where slanted transversal markings 51, 52 are at a constant non-right angle to the elastic bandage edge 20. The angle is chosen so that the transversal markings 51, 52 align in the longitudinal direction of the body part L, as the elastic bandage is stretched around a body part L with a specific circumference and a specific overlap. For other circumferences or overlaps, the markings 15, 16 will no longer align in the longitudinal direction of the body part, but the angle to the longitudinal direction will typically be smaller than in the embodiment in Fig. 3.

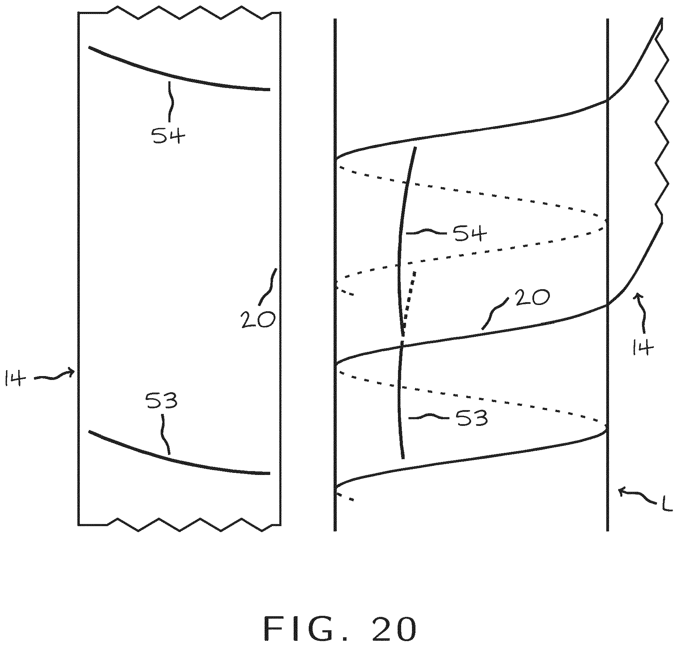

Fig. 20 shows an embodiment similar to that in Fig. 19, but where slanted and curved transversal markings 53, 54 are provided. The marking lines are here curved in such a way that the parts of 53, 54 that are closest to the edge 20 align in the longitudinal direction of the body part L when the elastic bandage is wrapped at any overlap around the body part with a specific circumference. For other circumferences, the parts of 53, 54 that are closest to the edge 20 will no longer align, but the distance between the parts will be smaller than in Fig. 3 and, for general overlaps, smaller than in Fig. 19.

The elastic bandage material having the above-discussed elastic and mechanical properties may be realized in many various ways, as would be obvious for the skilled addressee. In the following, some presently preferred realizations will be discussed in more detail.

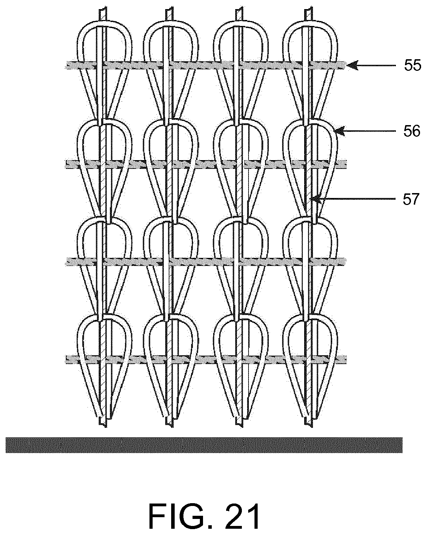

An elastic textile bandage as discussed above may be composed of a warp knitted construction, as illustrated schematically in Fig. 21, including both inelastic yarns - weft inlay 55 and warp (ground yarn) 56 - and elastic threads 57. The textile bandage in this example may be produced on a Standard Raschel warp knitting machine, with various numbers of warp bars on the knitting machine. The gauge (wales/inch) could be varied depending on the desired mesh size of the textile material and could range from E 8 to E 30 and preferably between E 10 and E 20.

The knitting machine preferably has thread guides for a weft inlay system over the whole fabric width. The thread guides inserts the weft threads in parallel with the machine's needle bed. The mesh forming warp system performs an open pillar stitch into which the elastic threads and the inelastic weft yarns are placed.

The warp (open pillar stitch) is knitted on every needle, one could also knit the open pillar stitch on every second needle to get a wider mesh or use a machine with a different gauge.

The preferred warp in this example may be a multifilament yarn of 100% polyester; one could also use a mixed spun yarn with multifilament polyester fibres and cotton or viscose fibres. The weft inlay is preferably a weave spun yarn, of 100% cotton. However, other fibre materials may also be used. For example, weave spun yarn of viscose or a mixed fibre yarn may also be used, for example cotton/viscose or cotton/polyester or viscose/polyester or other mixtures thereof. The elastic threads can be either monofilament or multifilament yarn, where monofilament is preferred as they are more suitable to use in the machine's inlay system. The elastic threads in the example that are inlayed in the warp direction are preferably elastomeric polymer monofilament threads. The elastomeric polymers could be of natural rubber, but preferably synthetic rubber is used.

In the exemplary embodiment, the elastic threads have a rectangular cross section, with dimensions preferably ranging from 0.5 milimeter to 1.0 millimeter. However, other dimensions may also be used. The cross section of the elastic threads could also be circular with different dimensions as mentioned above.

The elastic threads are preferably inlayed into the knitted structure under a certain elongation (tension). The weft inlay over the whole fabric width binds the whales together, causing less stiffness or impact on the stretch properties in the warp direction. The weft inlay can be inserted on both sides of the warp, using a fine yarn or inserted only on one side, using a thicker yarn. However the weft inlay gives a very form stable and a non-stretchable construction in the weft direction hence minimizing the impact on the elasticity in the warp direction, also the width decrease of the bandage in the longitudinal direction is very limited when stretched.

Each wale of open pillar stich preferably includes an elastic thread. The number of elastic threads/inch in the construction is hereby equal to the gauge. The mesh density (number of courses/cm) can be varied, and can for example range from 4 to 14 courses/cm, depending on the desired properties.

Another technique to produce an elastic textile bandage material is to use weaving. In a plain weave, the warp could include the elastic threads. Hereby, the elastic properties would be in the longitude direction as is commonly preferred in a bandage. The weft could be of the same material as described for the weft in the warp knitting technique. One could also wary the woven structure using plain weave with an interlacement at every thread, one over - one under, or the related variants such as: two over - two under, or three over - three under.

A technique to produce a tubular bandage or a compression sock could be weft knitting, using a circular knitting machine. To this end, it is possible to incorporate the elastic threads together with the yarn that forms the stitches, giving the tubular bandage elastic properties in both directions. The elastic threads could also be laid into the knitting tube as weft inlay, similar to the incorporation of the elastic threads in the warp knitting technique. As before, the ground yarn that performs the stitches could be either a spun multifilament yarn of cotton, polyester, viscose or a spun multifilament yarn with mixed fibres for example cotton/polyester, cotton/viscose, or polyester/viscose or other mixtures thereof.

Another example to make a tubular bandage or a sock could be to use warp knitting technique. This could be done either on a circular warp-knitting machine or a flat warp-knitting machine. By using a circular machine with a weft inlay system to incorporate the elastic treads in the ground stitches one could create a tubular bandage or a sock that corresponds to the above described criteria. Using a flat warp-knitting machine, the width could be extended by increasing the number of working needles, thereby producing a wider fabric that could be sewn together to form a tube. The seam could for example be a flatlock seam, avoiding seam allowance that could cause marks on the skin. Another technique instead of a seam could be welding, using either thermoplastic material in the yarns or by using a thermoplastic tape that would work as the adhesive between the two fabric surfaces that has to be welded together to form a tube.

The invention has now been disclosed by reference to preferred embodiments. However, it is to be acknowledged by the skilled addressee that several further modifications are feasible. For example, other elastic materials, and combinations of in-elastic and elastic materials may be used, other production technologies may be employed, etc. Further, the repeated markings allowing the turns to use a predetermined amount of unstretched elastic bandage material may be realized in many different ways, some of which have been disclosed in the foregoing. However, many other alternative embodiments would be feasible to the same or similar ends.

Such and other obvious modifications must be considered to be within the scope of the present invention, as it is defined by the appended claims. It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting to the claim. The word "comprising" does not exclude the presence of other elements or steps than those listed in the claim. The word "a" or "an" preceding an element does not exclude the presence of a plurality of such elements.

**Claims**

1. An elastic bandage (14) comprising an elongate strip of a stretchable elastic textile material, **characterized in that** a first set of repeated markings (15, 15a-c, 16, 18, 19, 21-25, 27, 28, 33-36, 47-49, 50a-c, 51-54) are provided and distributed along the longitudinal direction of the elongate strip for correlation of markings within different turns, allowing turns having the same length of unstretched elastic bandage to be wrapped and stretched around various circumferences with different yield rates, and wherein the elastic property of the elongate strip is such that the pressure exerted by such turns having the same length of unstretched elastic bandage varies less than 30% over a range of approximately circular circumferences providing a range of yield rates from $\lambda_1$ to $\lambda_2$, wherein $\lambda_2/\lambda_1 > 1.8$.

2. The elastic bandage of claim 1, wherein the pressure exerted by such turns having the same length of unstretched elastic bandage varies less than 20% over a range of circumferences providing a range of yield rates from $\lambda_1$ to $\lambda_2$, and preferably varies less than 10%

3. The elastic bandage of claim 1 or 2, wherein the $\lambda_2/\lambda_1 > 1.9$, and preferably $\lambda_2/\lambda_1 > 2.0$.

4. The elastic bandage of any one of the preceding claims, wherein the markings (15, 15a-c, 16, 18, 19, 21-25, 27, 28, 33-36, 47-49, 50a-c, 51-54) are visually and/or tactilely discernible.

5. The elastic bandage of any one of the preceding claims, wherein markings are also provided to determine the degree of overlap between different turns, said markings preferably being either the first set of markings arranged also to determine the degree of overlap between the different turns, or a second set of markings (39, 40, 41, 42, 43, 44, 45, 46) is provided to determine the degree of overlap between different turns, e.g. comprising markings arranged along or forming at least one line extending in the length direction of the elastic bandage.

6. The elastic bandage of any one of the preceding claims, wherein the first set of markings comprises marking lines extending at least partly in a transversal direction of the elastic bandage.

7. The elastic bandage of any one of the preceding claims, wherein the first set of markings comprises markings extending over more than 50% of the width of the elastic bandage.

8. The elastic bandage of any one of the claims 1-6, wherein the first set of markings comprises markings having a limited extension in the width direction of the elastic bandage, and being arranged in the vicinity of at least one of the longitudinal sides of the elastic bandage.

9. The elastic bandage of any one of the preceding claims, wherein the first set of markings comprises characters or digits forming a continuous or discontinuous scale in the length direction of the elastic bandage.

10. The elastic bandage of any one of the preceding claims, wherein the first set of markings comprises at least one set of markings which are repeatedly and equidistantly arranged in the lengthwise direction of the elastic bandage.

11. The elastic bandage of any one of the preceding claims,
wherein the first set of markings comprises at least one set of markings which are repeatedly and arranged at different distances from each other in the lengthwise direction of the elastic bandage.

12. The elastic bandage of any one of the preceding claims,
wherein the textile material comprises synthetic fibres selected from the group consisting of polyester, polyamide, polypropylene or PLA (polylactic acid), the textile material further preferably comprising natural fibres, such as cotton or regenerated fibres such as viscose or a mixed spun yarn with multifilament synthetic fibres and natural staple fibres or other mixtures thereof.

13. The elastic bandage of any one of the preceding claims,
wherein the textile material comprises threads or yarns of at least one elastic material, said elastic material comprising at least one of: elastomeric polymers such as natural rubber ( i.e. polyisoprene), synthetic rubber, a mix of polyisoprene rubber and styrene butadiene copolymer or a mix of thermoplastic and elastomeric polymers such as polyurethane-polyurea copolymer, and other mixtures thereof.


**Patentansprüche**

1. Elastische Bandage (14), umfassend einen länglichen Streifen eines dehnbaren elastischen Textilmaterials, **dadurch gekennzeichnet, dass** ein erster Satz sich wiederholender Markierungen (15, 15a-c, 16, 18, 19, 21-25, 27, 28, 33-36, 47-49, 50a-c, 51-54) entlang der Längsrichtung des länglichen Streifens zur Korrelation von Markierungen innerhalb unterschiedlicher Windungen vorgesehen und verteilt ist, was ermöglicht, dass Windungen, welche dieselbe Länge ungedehnter elastischer Bandage aufweisen, um verschiedene Umfänge mit unterschiedlichen Beaufschlagungsraten gewickelt und gedehnt werden, und wobei die elastische Eigenschaft des länglichen Streifens derart ist, dass der Druck, der durch derartige Windungen mit derselben Länge ungedehnter elastischer Bandage ausgeübt wird, über einen Bereich von ungefähr kreisförmigen Umfängen um weniger als 30% variiert, was einen Bereich von Beaufschlagungsraten von $\lambda_1$ bis $\lambda_2$ vorsieht, wobei $\lambda_2/\lambda_1 > 1{,}8$.

2. Elastische Bandage nach Anspruch 1, wobei der Druck, der durch derartige Windungen mit derselben Länge ungedehnter elastischer Bandage ausgeübt wird, über einen Bereich von Umfängen um weniger als 20% variiert, was einen Bereich von Beaufschlagungsraten von $\lambda_1$ bis $\lambda_2$ vorsieht, und vorzugsweise um weniger als 10% variiert.

3. Elastische Bandage nach Anspruch 1 oder 2, wobei $\lambda_2/\lambda_1 > 1{,}9$ und vorzugsweise $\lambda_2/\lambda_1 > 2{,}0$.

4. Elastische Bandage nach einem der vorhergehenden Ansprüche, wobei die Markierungen (15, 15a-c, 16, 18, 19, 21-25, 27, 28, 33-36, 47-49, 50a-c, 51-54) visuell und/oder taktil erkennbar sind.

5. Elastische Bandage nach einem der vorhergehenden Ansprüche, wobei Markierungen auch vorgesehen sind, um den Überlappungsgrad zwischen unterschiedlichen Windungen zu bestimmen, wobei die Markierungen vorzugsweise entweder der erste Satz Markierungen ist, der auch angeordnet ist, um den Überlappungsgrad zwischen den unterschiedlichen Windungen zu bestimmen, oder ein zweiter Satz Markierungen (39, 40, 41, 42, 43, 44, 45, 46), der vorgesehen ist, um den Überlappungsgrad zwischen unterschiedlichen Windungen zu bestimmen, der z. B. Markierungen umfasst, die entlang mindestens einer Linie angeordnet sind, die sich in der Längenrichtung der elastischen Bandage erstreckt, oder diese bilden.

6. Elastische Bandage nach einem der vorhergehenden Ansprüche, wobei der erste Satz Markierungen Markierungslinien umfasst, die sich zumindest teilweise in einer Querrichtung der elastischen Bandage erstrecken.

7. Elastische Bandage nach einem der vorhergehenden Ansprüche, wobei der erste Satz Markierungen Markierungen umfasst, die sich über mehr als 50% der Breite der elastischen Bandage erstrecken.

8. Elastische Bandage nach einem der Ansprüche 1-6, wobei der erste Satz Markierungen Markierungen umfasst, die eine begrenzte Erstreckung in der Breitenrichtung der elastischen Bandage aufweisen und in der Nähe mindestens einer der Längsseiten der elastischen Bandage angeordnet sind.

9. Elastische Bandage nach einem der vorhergehenden Ansprüche, wobei der erste Satz Markierungen Buchstaben oder Ziffern umfasst, die eine kontinuierliche oder diskontinuierliche Skala in der Längenrichtung der elastischen Bandage bilden.

10. Elastische Bandage nach einem der vorhergehenden Ansprüche, wobei der erste Satz Markierungen mindestens einen Satz Markierungen umfasst, die wiederholt und äquidistant in der Längsrichtung der elastischen Bandage angeordnet sind.

11. Elastische Bandage nach einem der vorhergehenden Ansprüche, wobei der erste Satz Markierungen mindestens einen Satz Markierungen umfasst, die wiederholt und in unterschiedlichen Abständen voneinander in der Längsrichtung der elastischen Bandage angeordnet sind.

12. Elastische Bandage nach einem der vorhergehenden Ansprüche, wobei das Textilmaterial Kunstfasern umfasst, die aus der Gruppe ausgewählt sind, die aus Polyester, Polyamid, Polypropylen oder PLA (Polymilchsäure) besteht, wobei das Textilmaterial ferner vorzugsweise Naturfasern, wie etwa Baumwoll- oder Regeneratfasern, wie etwa Viskose oder einen gemischten Spinngarn mit Multifil-Kunstfasern und natürlichen Stapelfasern, oder Gemische davon umfasst.

13. Elastische Bandage nach einem der vorhergehenden Ansprüche, wobei das Textilmaterial Fäden oder Garne aus mindestens einem elastischen Material umfasst, wobei das elastische Material mindestens eines umfasst aus: elastomeren Polymeren, wie etwa Naturkautschuk (d. h. Polyisopren), Synthesekautschuk, einem Gemisch aus Polyisoprenkautschuk und Styrol-Butadien-Copolymer oder einem Gemisch aus thermoplastischen und elastomeren Polymeren, wie etwa Polyurethan-Polyharnstoff-Copolymer, und anderen Gemische davon.


**Revendications**

1. Bande élastique (14) comprenant un ruban allongé d'une matière textile élastique extensible, **caractérisée en ce qu'**un premier ensemble de marquages répétés (15, 15a-c, 16, 18, 19, 21-25, 27, 28, 33-36, 47-49, 50a-c, 51-54) sont disposés et répartis le long de la direction longitudinale du ruban allongé pour la mise en corrélation de marquages dans différentes spires, de manière à ce que des spires présentent la même longueur de bande élastique non étirée puissent être enroulées et étirées autour de diverses circonférences avec différents taux de rendement, et dans laquelle la propriété élastique du ruban allongé est telle que la pression exercée par de telles spires présentant la même longueur de bande élastique non étirée varie de moins de 30% sur une plage de circonférences approximativement circulaires fournissant une plage de taux de rendement de $\lambda_1$ à $\lambda_2$, où $\lambda_2/\lambda_1 > 1,8$.

2. Bande élastique selon la revendication 1, dans laquelle la pression exercée par de telles spires présentant la même longueur de bande élastique non étirée varie de moins de 20% sur une plage de circonférences approximativement circulaires fournissant une plage de taux de rendement de $\lambda_1$ à $\lambda_2$, et varie de préférence de moins de 10%.

3. Bande élastique selon la revendication 1 ou 2, dans laquelle $\lambda_2/\lambda_1 > 1,9$, et de préférence $\lambda_2/\lambda_1 > 2,0$.

4. Bande élastique selon l'une quelconque des revendications précédentes, dans laquelle les marquages (15, 15a-c, 16, 18, 19, 21-25, 27, 28, 33-36, 47-49, 50a-c, 51-54) sont discernables visuellement et/ tactilement.

5. Bande élastique selon l'une quelconque des revendications précédentes, dans laquelle des marquages sont également prévus pour déterminer le degré de chevauchement entre différentes spires, lesdits marquages étant de préférence soit le premier ensemble de marquages également conçu pour déterminer le degré de chevauchement entre les différentes spires, soit un deuxième ensemble de marquages (39, 40, 41, 42, 43, 44, 45, 46) prévu pour déterminer le degré de chevauchement entre différentes spires, comprenant par exemple des marquages disposés le long d'une ligne ou formant au moins une ligne s'étendant dans la direction longitudinale de la bande élastique.

6. Bande élastique selon l'une quelconque des revendications précédentes, dans laquelle le premier ensemble de marquages comprend des lignes de marquages s'étendant au moins partiellement dans une direction transversale de la bande élastique.

7. Bande élastique selon l'une quelconque des revendications précédentes, dans laquelle le premier ensemble de marquages comprend des marquages s'étendant sur plus de 50% de la largeur de la bande élastique.

8. Bande élastique selon l'une quelconque des revendications 1 à 6, dans laquelle le premier ensemble de marquages comprend des marquages présentant une extension limitée dans la direction de la largeur de la bande élastique, tout en étant disposés à proximité de l'un au moins des côtés longitudinaux de la bande élastique.

9. Bande élastique selon l'une quelconque des revendications précédentes, dans laquelle le premier ensemble de marquages comprend des lettres ou des chiffres formant une échelle continue ou discontinue dans la direction longitudinale de la bande élastique.

10. Bande élastique selon l'une quelconque des revendications précédentes, dans laquelle le premier ensemble de marquages comprend au moins un ensemble de marquages disposés de façon répétée et équidistante dans la direction longitudinale de la bande élastique.

11. Bande élastique selon l'une quelconque des revendications précédentes, dans laquelle le premier ensemble de marquages comprend au moins un ensemble de marquages répétés et disposés à des distances différentes les uns des autres dans la direction longitudinale de la bande élastique.

12. Bande élastique selon l'une quelconque des revendications précédentes, dans laquelle la matière textile comprend des fibres synthétiques sélectionnées à partir du groupe constitué du polyester, du polyamide, le polypropylène ou le PLA (acide polylactique), la matière textile comprenant de préférence également des fibres naturelles, telles que le coton ou des fibres régénérées telles que la viscose ou un fil filé mixte avec des fibres synthétiques multi-filament et des fibres naturelles discontinues ou des mélanges de ceux-ci.

13. Bande élastique selon l'une quelconque des revendications précédentes, dans laquelle la matière textile comprend des fils constitués d'au moins une matière élastique, ladite matière élastique comprend l'un au moins parmi : des polymères élastomères tels que le caoutchouc naturel (c'est-à-dire le polyisoprène), le caoutchouc synthétique, un mélange de caoutchouc de polyisoprène et de copolymère de butadiène styrène ou un mélange de polymères thermoplastiques ou élastomères tels qu'un copolymère de polyuréthane-polyurée, et d'autres mélanges de ceux-ci.

FIG. 1

FIG. 2

FIG. 3

FIG. 4

FIG. 5

EP 3 021 805 B1

FIG. 6

21

FIG. 7

FIG. 8

FIG. 9

FIG. 10

FIG. 11

FIG. 12

FIG. 13

FIG. 14

FIG. 15

FIG. 16

FIG. 17

FIG. 18

FIG. 19

# FIG. 20

55

56

57

# FIG. 21

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008152294 A
- US 5779659 A
- US 5749843 A
- US 3613679 A
- US 2009099497 A
- GB 2104558 A
- US 6050967 A
- WO 9847452 A
- US 5195950 A
- US 6338723 B
- WO 03005945 A
- US 6415525 B